# EUROPÄISCHE PATENTSCHRIFT

(11) **EP 2 525 750 B1**
(45) Veröffentlichungstag und Bekanntmachung des Hinweises auf die Patenterteilung: **28.06.2017**
(21) Anmeldenummer: 10702044.8
(22) Anmeldetag: 22.01.2010
(51) Int. Cl.: A61F 9/008, A61F 9/009

(54) **EINRICHTUNG ZUR SCHNEIDENDEN BEARBEITUNG DER HUMANEN KORNEA**
DEVICE FOR CUTTING THE HUMAN CORNEA
APPAREIL POUR LE TRAITEMENT PAR DÉCOUPE DE LA CORNÉE HUMAINE

(43) Veröffentlichungstag der Anmeldung: 28.11.2012
(73) Patentinhaber: WaveLight GmbH, 91058 Erlangen (DE)
(72) Erfinder: DONITZKY, Christof, 90542 Eckental/Eschenau (DE); WÖLFEL, Mathias, 91052 Erlangen (DE)
(74) Vertreter: Katérle, Axel
(86) Internationale Anmeldenummer: PCT/EP2010/000393
(87) Internationale Veröffentlichungsnummer: WO 2011/088848

(56) Entgegenhaltungen:
- EP-A2- 1 034 757
- WO-A1-2009/152838
- US-A1- 2003 212 387
- US-A1- 2004 243 111
- US-A1- 2008 319 428

## Beschreibung

Die Erfindung befasst sich mit der Erzeugung von Schnitten in der humanen Kornea mittels fokussierter gepulster Laserstrahlung. Insbesondere befasst sich die Erfindung mit der Präparation eines LASIK-Flaps mittels solcher Laserstrahlung.

Eine häufig eingesetzte Technik zur Behebung von Fehlsichtigkeiten des menschlichen Auges, wie beispielsweise Kurz- oder Weitsichtigkeit oder Astigmatismus, ist die sogenannte LASIK. LASIK steht für Laser In-situ Keratomileusis und bezeichnet eine Technik, bei der zunächst ein Deckelscheibchen in der Kornea freigeschnitten wird, das zur Seite geklappt wird, um die darunterliegenden Gewebebereiche der Kornea freizulegen. Diese freigelegten Gewebebereiche werden sodann mittels fokussierter UV-Laserstrahlung ablatierend behandelt, d.h. es wird nach Maßgabe eines individuell für den Patienten ermittelten Ablationsprofils korneales Material abgetragen. Das Deckelscheibchen wird in der Fachwelt regelmäßig als Flap bezeichnet und wird nicht vollständig vom Restgewebe der Kornea getrennt, sondern hängt noch in einem Scharnierbereich, der in der Fachwelt gemeinhin als Hinge bezeichnet wird, mit dem übrigen kornealen Gewebe zusammen. Dies ermöglicht ein einfaches Wegklappen des Flaps und vor allem ein einfaches Zurückklappen des Flaps nach der Ablation. Wegen des Materialabtrags stellt sich nach Zurückklappen des Flaps eine veränderte Form der Korneavorderfläche ein. Damit einhergehend ergibt sich ein anderes Brechungsverhalten der Kornea und folglich des Gesamtsystems Auge. Durch geeignete Festlegung des Ablationsprofils kann erreicht werden, dass die Fehlsichtigkeit zumindest deutlich abgeschwächt wird und bestenfalls sogar nahezu vollständig behoben wird.

Für die Präparation des Flaps sind im Stand der Technik verschiedene Vorgehensweisen bekannt. Eine Vorgehensweise nutzt ein mechanisches Mikrokeratom, d.h. ein mikrochirurgisches Skalpell, das mit einer üblicherweise oszillierend angetriebenen Schneidklinge in die Kornea einschneidet. Eine andere Vorgehensweise, die im Rahmen der Erfindung näher betrachtet wird, nutzt fokussierte kurzpulsige Laserstrahlung zur Präparation des Flaps. üblicherweise wird hierbei Laserstrahlung mit Pulsdauern im Femtosekundenbereich, beispielsweise im niederen dreistelligen Femtosekundenbereich, eingesetzt. Die Laserstrahlung besitzt zudem regelmäßig eine Wellenlänge oberhalb ca. 300 nm, um eine Einkopplung der Strahlungsenergie in die Tiefe des kornealen Gewebes zu ermöglichen. LASIK-Behandlungen, bei denen der Flap mittels solcher kurzpulsiger Laserstrahlung präpariert wird, werden oftmals als Fs-LASIK bezeichnet.

Für die Erzeugung von Schnitten mittels fokussierter Laserstrahlung in transparentem Material (transparent für die Laserstrahlung) wird als physikalischer Effekt der sogenannte laserinduzierte optische Durchbruch genutzt. Dieser führt letztendlich zu einer Photodisruption des bestrahlten Gewebes im Bereich des Fokus. Die eingestrahlte Laserstrahlung bewirkt im Fokuspunkt eine lokale Verdampfung des bestrahlten Materials. Dabei entstehen Gase, die - soweit sie nicht nach außen abgeführt werden - sich in internen Hohlräumen sammeln oder vom angrenzenden Material aufgenommen werden. Es hat sich gezeigt, dass bei LASIK-Behandlungen des humanen Auges ein Verbleib der bei der Flap-Präparation entstehenden Gase in der Kornea zu Problemen bei der anschließenden Laserablation führen kann. Insbesondere hat sich gezeigt, dass diese Gase eine präzise Verfolgung des Auges mittels eines Eye-Trackers erschweren können. Lasersysteme, die für die Ablation kornealen Gewebes eingesetzt werden, besitzen häufig einen solchen Eye-Tracker, um während der Laserbehandlung Augenbewegungen zu erfassen und die Laserstrahlung entsprechend nachzuführen. In der Regel sind die Eye-Tracker aus einer Kamera und einer geeigneten Bildauswertungssoftware aufgebaut, welche die von der Kamera aufgenommenen Bilder auswertet und Veränderungen der Augenposition erkennt. Häufig wertet die Bildauswertungssoftware charakteristische Merkmale des Auges aus, etwa bestimmte Punkte der Iris oder/und das Pupillenzentrum oder/und den Korneaapex oder/und den Limbus. Es hat sich gezeigt, dass in der Kornea verbleibende Gasansammlungen, die im Zuge der Flap-Präparation entstanden sind, die Erfassung solcher charakteristischen Merkmale des Auges behindern kann. Es versteht sich von selbst, dass für den Operationserfolg eine präzise Funktion des Eye-Trackers unbedingt vonnöten ist.

Aufgabe der Erfindung ist es, einen Weg aufzuzeigen, wie bei LASIK-Behandlungen eine Beeinträchtigung des Operationserfolgs durch störende Gasansammlungen, die im Zuge einer lasertechnischen Präparation des LASIK-Flaps entstehen, vermieden werden können. Alternative Lösungen sind zum Beispiel in US 2003/0212387 beschrieben, wobei ein Hilfsreservoir erzeugt wird, mit natürlichen oder durch Laser erzeugten Kanälen in Kontakt zum Flapschnitt, durch die die Gase in das Reservoir geleitet werden. Zur Lösung dieser Aufgabe schlägt die Erfindung eine Einrichtung zur schneidenden Bearbeitung der humanen Kornea mit fokussierter gepulster Laserstrahlung vor. Die Einrichtung umfasst steuerbare Komponenten zur Ortseinstellung des Strahlfokus, einen Steuerrechner zur Steuerung dieser Komponenten und ein Steuerprogramm für den Steuerrechner. Das Steuerprogramm enthält Instruktionen, welche dazu ausgelegt sind, bei Ausführung durch den Steuerrechner die Erzeugung einer einen Flapschnitt umfassenden Schnittfigur in der Kornea zu bewirken, wobei gemäß der Erfindung die Schnittfigur ferner einen mit dem Flapschnitt in Verbindung stehenden, örtlich bis zur Korneaoberfläche führenden Hilfsschnitt umfasst. Der Hilfsschnitt stellt eine Verbindung zwischen dem Flapschnitt und der Hornhautoberfläche her. Auf diese Weise können operativ entstehende Gase zur Hornhautoberfläche und damit aus dem kornealen Gewebe entweichen. Ein Eindringen dieser Gase in kritische Gewebebereiche des Auges kann so vermieden werden. Auch können etwaige Beeinträchtigungen eines Eye-Trackers bei der nachfolgenden Laserablation besser vermieden werden.

Der obige Hinweis auf einen örtlichen Verlauf des Hilfsschnitts soll dazu dienen, Verwechslungen mit dem zeitlichen Verlauf bei der Erzeugung des Hilfsschnitts zu vermeiden. Eine Angabe über einen bestimmten örtlichen Verlauf eines Schnitts oder eines Teils eines Schnitts soll hier generell keinerlei Aussage über die zeitliche Abfolge bei der Erzeugung des Schnitts beinhalten. So kann ein Schnitt, für den die Aussage gemacht wird, dass er örtlich von einem bestimmten ersten Ort zu einem bestimmten zweiten Ort verläuft, ohne weiteres zeitlich in Richtung von dem zweiten Ort zu dem ersten Ort erzeugt werden.

In einer bevorzugten Ausgestaltung geht der Hilfsschnitt örtlich unmittelbar von dem Flapschnitt aus und verläuft örtlich weg von diesem bis zur Korneaoberfläche. Es ist freilich auch vorstellbar, dass angrenzend an den Flapschnitt zunächst eine oder mehrere Gastaschen oder -hohlräume innerhalb des kornealen Gewebes operativ erzeugt werden und dass der Hilfsschnitt von diesen Gastaschen bzw. -hohlräumen örtlich zur Korneaoberfläche führt.

Bevorzugt bildet der Hilfsschnitt einen flächigen und gewünschtenfalls im wesentlichen ebenen Kanal, wobei die Länge, die Breite und der Steigungswinkel des Kanals variabel gewählt werden können. Der Kanal kann über seine Länge im wesentlichen gleichbleibende Breite besitzen; es soll freilich nicht ausgeschlossen sein, einen Kanal variierender Breite zu erzeugen. Für die Erzeugung des Hilfsschnitts kann es genügen, Photodisruptionen nur in einer einzigen Ebene nebeneinander zu setzen. Es soll freilich nicht ausgeschlossen sein, solche Photodisruptionen auch in zwei oder mehr Ebenen übereinander zu setzen, wenn ein größerer Kanalquerschnitt erwünscht ist.

In einer bevorzugten Ausgestaltung steht der Hilfsschnitt in einem Scharnierbereich (Hinge) des durch den Flapschnitt gebildeten Flaps mit dem Flapschnitt in Verbindung und erstreckt sich jenseits des Flaps örtlich zur Korneaoberfläche hin. Gewünschtenfalls ist dabei der Hilfsschnitt schmaler als der Scharnierbereich.

Für eine bestmögliche Abfuhr entstehender Photodisruptionsgase (d.h. Gase, die bei und infolge der Photodisruption entstehen) empfiehlt es sich, dass der Hilfsschnitt in einem Bereich, in dem er in Verbindung mit dem Flapschnitt steht, größte korneale Tiefe besitzt und örtlich ausgehend von diesem Bereich in zunehmend geringerer kornealer Tiefe bis hin zur Korneaoberfläche verläuft.

Günstig ist es, wenn die Instruktionen des Steuerprogramms dazu ausgelegt sind, den Hilfsschnitt anzubringen, bevor der Flapschnitt gesetzt wird. Dies schafft die Voraussetzung dafür, dass von Anfang an ein Gasabführkanal bereitsteht, über den die bei der Flap-Präparation entstehenden Photodisruptionsgase abgeführt werden können.

Vorteilhafterweise sind die Instruktionen des Steuerprogramms dazu ausgelegt, zumindest einen überwiegenden und bevorzugt den größten Teil des Hilfsschnitts in einer Richtung von der Korneaoberfläche hin zum Flapschnitt zu erzeugen. Auch bei der Erzeugung des Hilfsschnitts ist mit dem Entstehen von Photodisruptionsgasen zu rechnen. Indem der Hilfsschnitt in Richtung von der Korneaoberfläche weg erzeugt wird, gelingt es, diese Photodisruptionsgase bestmöglich abzuführen. Beispielsweise können die Instruktionen des Steuerprogramms dazu ausgelegt sein, zumindest einen überwiegenden Teil des Hilfsschnitts mit Linienscans des Strahlfokus zu erzeugen, welche Linie für Linie längs einer Erstreckungsrichtung des Hilfsschnitts voranschreiten, die von der Korneaoberfläche zum Flapschnitt verläuft. Es ist freilich ebenso möglich, dass die Instruktionen des Steuerprogramms dazu ausgelegt sind, zumindest einen überwiegenden Teil des Hilfsschnitts mit Linienscans des Strahlfokus zu erzeugen, welche Linie für Linie quer zu einer Erstreckungsrichtung des Hilfsschnitts voranschreiten, die von der Korneaoberfläche zum Flapschnitt verläuft.

Der Flapschnitt kann einen vollständig in der Tiefe, vorzugsweise in im wesentlichen gleichbleibender Tiefe, des Korneamaterials liegenden Bettschnitt sowie einen an den Bettschnitt angrenzenden, örtlich zur Korneaoberfläche herausgeführten Seitenschnitt umfassen. Der Bettschnitt wird so bezeichnet, weil er das stromale Bett für den Flap definiert. Bei vorheriger Applanation (Einebnung) der Korneaoberfläche durch Anlage an einer geeigneten Applanationsfläche kann der Bettschnitt beispielsweise durch einen ebenen Flächenschnitt realisiert werden, der in konstanter Tiefe der Kornea angebracht wird. Grundsätzlich ist es vorstellbar, den Bettschnitt mit Linienscans oder mit Spiralscans des Strahlungsfokus zu erzeugen. Für eine bestmögliche Abfuhr der bei der Erzeugung des Bettschnitts entstehenden Photodisruptionsgase wird jedoch vorgeschlagen, dass die Instruktionen des Steuerprogramms dazu ausgelegt sind, den Bettschnitt mit Linienscans des Strahlungsfokus zu erzeugen, welche Linie für Linie zunehmend in Richtung weg von einem Scharnierbereich (Hinge) des Flaps voranschreiten. Zugleich sind die Instruktionen des Steuerprogramms zweckmäßigerweise dazu ausgelegt, den Seitenschnitt zeitlich nach dem Bettschnitt anzubringen. Da der Seitenschnitt örtlich zur Korneaoberfläche herausführt, empfiehlt es sich, den Seitenschnitt ausgehend von seinen tiefstliegenden Bereichen in fortschreitender Richtung hin zur Korneaoberfläche zu erzeugen. Selbstverständlich ist eine umgekehrte Richtung bei der Erzeugung des Seitenschnitts gleichermaßen vorstellbar.

Für einen guten, für die Photodisruptionsgase ausreichend durchlässigen Übergang zwischen dem Hilfsschnitt und dem Flapschnitt empfiehlt es sich, dass die Instruktionen des Steuerprogramms dazu ausgelegt sind, in einem Übergangsbereich zwischen Hilfsschnitt und Flapschnitt Linienscans des Strahlfokus zu bewirken, welche Linie für Linie quer zur Übergangsrichtung voranschreiten. Gleichermaßen empfiehlt es sich für eine gute Öffnung des Hilfsschnitts nach außen, dass die Instruktionen des Steuerprogramms dazu ausgelegt sind, im oberflächenseitigen Endbereich des Hilfsschnitts (mit Endbereich ist hier ein örtlicher Endbereich gemeint; der oberflächenseitige Endbereich kann durchaus der Startbereich bei der Erzeugung des Hilfsschnitts sein) Linienscans des Strahlungsfokus zu bewirken, welche Linie für Linie quer zur Eintrittsrichtung des Hilfsschnitts in die Kornea voranschreiten.

In einer Weiterbildung der Erfindung kann die Einrichtung ferner ein für die Laserstrahlung transparentes Kontaktelement mit einer zur Anlage am Auge bestimmten Kontaktfläche umfassen, wobei die Instruktionen des Steuerprogramms dazu ausgelegt sind, den Hilfsschnitt so zu erzeugen, dass sein an der Korneaoberfläche ausmündendes Ende in einem Bereich der Kornea liegt, in dem diese an der Kontaktfläche des Kontaktelements anliegt.

In einer ersten Ausgestaltung kann dabei die Kontaktfläche einen planen Flächenabschnitt zur Einebnung eines Teils der Korneaoberfläche aufweisen, wobei die Instruktionen des Steuerprogramms dazu ausgelegt sind, den Hilfsschnitt so zu erzeugen, dass sein an der Korneaoberfläche ausmündendes Ende in einem Bereich der Kornea liegt, in dem diese an dem planen Flächenabschnitt der Kontaktfläche anliegt.

In einer alternativen Ausgestaltung kann die Kontaktfläche einen planen Flächenabschnitt zur Einebnung eines Teils der Korneaoberfläche sowie einen an den planen Flächenabschnitt angrenzenden, schräg zu diesem in Richtung zur augenabgewandten Seite des Kontaktelements verlaufenden Flächenabschnitt aufweisen, wobei die Instruktionen des Steuerprogramms dazu ausgelegt sind, den Hilfsschnitt so zu erzeugen, dass sein an der Korneaoberfläche ausmündendes Ende in einem Bereich der Kornea liegt, in dem diese an dem schräg verlaufenden Flächenabschnitt der Kontaktfläche anliegt. Der schräg verlaufende Flächenabschnitt ist vorzugsweise gerundet ausgeführt und schließt knickfrei an den planen Flächenabschnitt an.

Gemäß einer anderen Weiterbildung kann die Einrichtung ein für die Laserstrahlung transparentes Kontaktelement mit einer zur Anlage am Auge bestimmten Kontaktfläche umfassen, wobei die Instruktionen des Steuerprogramms dazu ausgelegt sind, den Hilfsschnitt so zu erzeugen, dass sein an der Korneaoberfläche ausmündendes Ende außerhalb eines Bereichs der Kornea liegt, in dem diese an der Kontaktfläche des Kontaktelements anliegt.

Bei dieser Weiterbildung ist auf der augenzugewandten Seite des Kontaktelements zur Anpassung des Brechungsindex vorzugsweise ein Raum vorgesehen, der über eine Füllkanalanordnung mit einer Flüssigkeit oder einem anderen geeigneten fließfähigen Medium befüllbar ist, welches den Brechungsindexsprung vom Kontaktelement zur Kornea reduziert und zweckmäßigerweise optisch homogene Eigenschaften besitzt. Dieses Medium kann statt in flüssiger Form beispielsweise auch in gelartiger Form vorliegen. Nicht ausgeschlossen ist auch die Verwendung eines gasförmigen Mediums. Die Instruktionen des Steuerprogramms sind dabei dazu ausgelegt, den Hilfsschnitt so zu erzeugen, dass er in den flüssigkeitsgefüllten (oder allgemein: in den mit dem Medium gefüllten) Raum mündet.

Bei Ausbildung des Flapschnitts mit einem ebenen Bettschnitt sind die Instruktionen des Steuerprogramms vorzugsweise dazu ausgelegt, den in den Raum ausmündenden Hilfsschnitt und den Bettschnitt in einer gemeinsamen Ebene zu erzeugen. Dies bezieht sich auf den applanierten Fall, also den Zustand, in dem das Auge an dem Kontaktelement anliegt.

Ein Verfahren zur Behandlung des humanen Auges umfasst die Schritte: Bereitstellen erster gepulster Laserstrahlung, wobei die Laserstrahlung einen Strahlungsfokus besitzt, Richten der ersten Laserstrahlung auf eine zu behandelnde humane Kornea, Steuern des Strahlungsfokus der ersten Laserstrahlung zur Erzeugung eines einen Flap bildenden Flapschnitts in der Kornea sowie eines mit dem Flapschnitt in Verbindung stehenden, örtlich bis zur Korneaoberfläche führenden Hilfsschnitts.

Das Verfahren kann ferner die Schritte umfassen: Wegklappen des Flaps, um dadurch darunterliegendes korneales Gewebe freizulegen, Bereitstellen zweiter gepulster Laserstrahlung, Richten der zweiten Laserstrahlung auf das freigelegte korneale Gewebe, und Ablatieren des freigelegten kornealen Gewebes mit der zweiten Laserstrahlung nach Maßgabe eines vorgegebenen Ablationsprofils.

Die erste Laserstrahlung besitzt vorzugsweise Pulsdauern im Femtosekundenbereich und hat eine Wellenlänge oberhalb 300 nm. Die zweite Laserstrahlung besitzt eine Wellenlänge im UV-Bereich und kann beispielsweise von einem Excimerlaser erzeugt werden, etwa einem bei 193 nm strahlenden ArF-Excimerlaser.

Die Erfindung wird nachfolgend anhand der beigefügten Zeichnungen weiter erläutert. Es stellen dar:
- Figur 1: in schematischer Blockdarstellung ein Ausführungsbeispiel einer Laseran-ordnung zur Anbringung intrakornealer Schnitte,
- Figuren 2a und 2b: zwei Varianten einer kornealen Schnittfigur zur Präparation eines LASIK-Flaps,
- Figur 3: die Schnittfigur der Figur 2a in einer Querschnittsansicht,
- Figur 4: ein beispielhaftes Scanmuster eines zur Erzeugung der Schnittfigur der Figur 2a verwendeten Laserstrahls,
- Figuren 5 und 6: zwei Varianten zur Erzeugung eines durch einen Hilfsschnitt ergänzten Flapschnitts in einer applanierten Kornea.

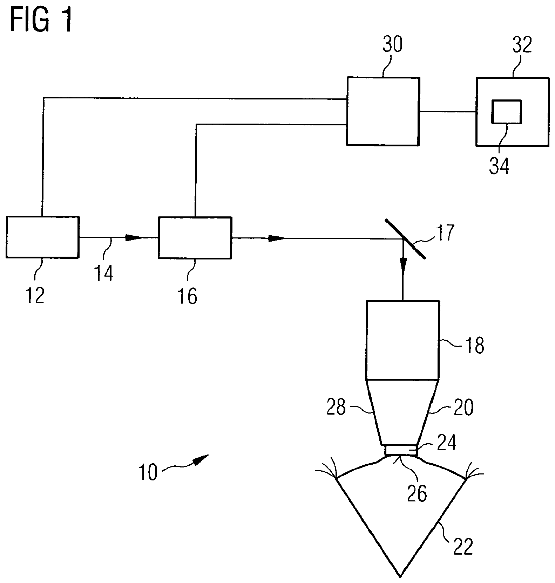

Die in Figur 1 gezeigte Laseranordnung - allgemein mit 10 bezeichnet - umfasst eine Laserquelle 12, welche einen Laserstrahl 14 mit Pulsdauern im Femtosekundenbereich erzeugt. Im Strahlengang des Laserstrahls 14 sind eine Reihe von Komponenten angeordnet, unter anderem ein hier schematisch als einheitlicher Funktionsblock angedeuteter Scanner 16, ein unbeweglicher Umlenkspiegel 17 sowie ein Fokussierobjektiv 18. Der Scanner 16 dient zur transversalen und longitudinalen Ortssteuerung des Fokuspunkts des Laserstrahls 14. Transversal bezeichnet hier eine Richtung quer zur Ausbreitungsrichtung des Laserstrahls 14, longitudinal entspricht der Strahlausbreitungsrichtung. In üblicher Notation wird die Transversalebene als x-y-Ebene bezeichnet, während die Longitudinalrichtung als z-Richtung bezeichnet wird. Zur Transversalablenkung des Laserstrahls 14 (d.h. in der x-y-Ebene) kann der Scanner 16 beispielsweise ein Paar galvanometrisch betätigter Scannerspiegel umfassen, die um zueinander senkrechte Achsen kippbar sind. Alternativ ist beispielsweise eine Transversalablenkung mittels eines elektrooptischen Kristalls denkbar. Für die z-Steuerung der Fokusposition kann der Scanner 16 beispielsweise eine longitudinal verstellbare oder brechkraftvariable Linse oder einen deformierbaren Spiegel enthalten, mit der bzw. dem sich die Divergenz des Laserstrahls 14 und folglich die z-Position des Strahlfokus beeinflussen lässt. Es versteht sich, dass die für die transversale Fokussteuerung und die longitudinale Fokussteuerung dienenden Komponenten des Scanners 16 entlang des Strahlengangs des Laserstrahls 14 verteilt angeordnet und insbesondere auf unterschiedliche Baueinheiten aufgeteilt sein können. Beispielsweise kann die Funktion der z-Fokussteuerung von einer in einer Strahlaufweitungsoptik (Beam Expander, z.B. Galileo-Teleskop) angeordneten Linse ausgefüllt werden, während die für die transversale Fokussteuerung dienenden Komponenten in einer gesonderten Baueinheit zwischen der Strahlaufweitungsoptik und dem Fokussierobjektiv 18 untergebracht sein können. Die Darstellung des Scanners 16 als einheitlicher Funktionsblock in Figur 1 dient lediglich der besseren Übersichtlichkeit.

Das Fokussierobjektiv 18 ist bevorzugt ein F-Theta-Objektiv und ist auf seiner Strahlaustrittsseite mit einem Patientenadapter 20 vorzugsweise lösbar gekoppelt, welcher eine Anlageschnittstelle für die Kornea eines zu behandelnden Auges 22 bildet. Der Patientenadapter 20 weist hierzu ein für die Laserstrahlung transparentes Kontaktelement 24 auf, welches auf seiner augenzugewandten Unterseite eine Anlagefläche (Kontaktfläche) 26 für die Kornea aufweist. Die Anlagefläche 26 ist im gezeigten Beispielfall als Planfläche ausgeführt und dient zur Einebnung der Kornea, indem das Kontaktelement 24 mit entsprechendem Druck gegen das Auge 22 gedrückt wird oder die Kornea durch Unterdruck an die Kontaktfläche 26 angesaugt wird. Das Kontaktelement 24 (bei planparalleler Ausführung üblicherweise als Applanationsplatte bezeichnet) ist am schmäleren Ende einer sich konisch aufweitenden Trägerhülse 28 angebracht. Die Verbindung zwischen dem Kontaktelement 24 und der Trägerhülse 28 kann unlösbar sein, beispielsweise durch Verklebung, oder sie kann lösbar sein, etwa durch eine Verschraubung. Die Trägerhülse 28 besitzt in nicht näher dargestellter Weise an ihrem breiteren Hülsenende geeignete Koppelformationen zur Ankopplung an das Fokussierobjektiv 18.

Die Laserquelle 12 und der Scanner 16 werden von einem Steuerrechner 30 gesteuert, der nach Maßgabe eines in einem Speicher 32 gespeicherten Steuerprogramms 34 arbeitet. Das Steuerprogramm 34 enthält Instruktionen (Programmcode), welche bei Ausführung durch den Steuerrechner 30 eine solche Ortssteuerung des Strahlfokus des Laserstrahls 14 bewirken, dass in der Kornea des an dem Kontaktelement 24 anliegenden Auges 22 ein LASIK-Flap entsteht. Die diesbezüglich in der Kornea erzeugte Schnittfigur umfasst nicht nur einen den eigentlichen Flap bildenden Flapschnitt, sondern zusätzlich einen Hilfsschnitt, durch den entstehende Photodisruptionsgase aus der Kornea nach außen entweichen können.

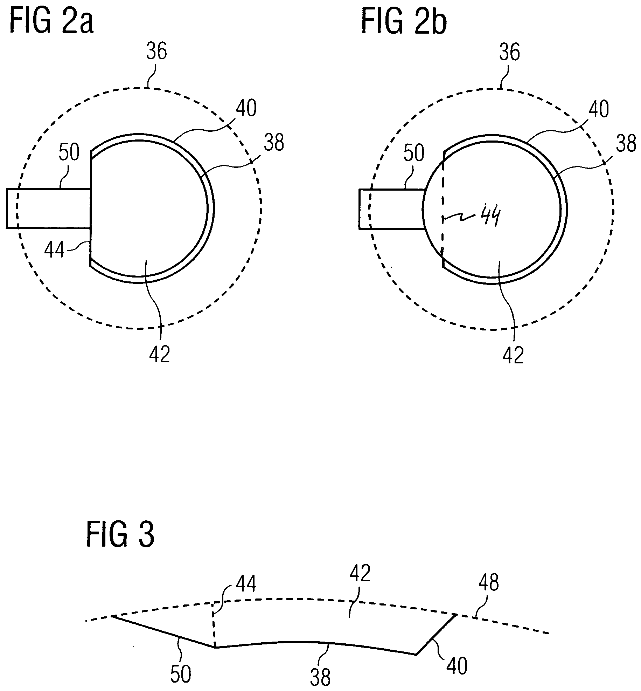

Die Figuren 2a und 2b zeigen zwei Varianten einer solchen Schnittfigur. In beiden Fällen bezeichnet eine gestrichelte Kreislinie 36 den Einebnungsbereich, in dem die Kornea infolge ihrer Anlage an dem Kontaktelement 24 eingeebnet ist. Es versteht sich, dass in der Realität der Einebnungsbereich 36 nicht exakt kreisrund sein muss. Insbesondere angesichts der üblicherweise verschiedenen Krümmungsradien in den Hauptmeridianrichtungen der Korneaoberfläche kann sich ein von einer Kreisform abweichender Umriss des Einebnungsbereichs 36 einstellen.

Der den Flap bildende Flapschnitt setzt sich in den gezeigten Beispielfällen aus zwei Teilschnitten zusammen. Ein erster Teilschnitt ist ein sogenannter Bettschnitt, der den Flap vom stromalen Bett trennt und als zur Kontaktfläche 26 paralleler ebener Flächenschnitt realisiert wird. Der Bettschnitt ist in den Figuren 2a und 2b mit 38 bezeichnet. Er wird in einer der gewünschten Flapdicke entsprechenden Tiefe der Kornea gesetzt. Während er sich in Figur 2b über eine vollständige Kreisfläche erstreckt, ist er in Figur 2a um einen Kreisabschnitt (Segment) verkürzt und endet an einer Kreissehne. Es versteht sich, dass je nach gewünschter Flapform der Bettschnitt 38 einen kreisfremden Umriss, beispielsweise einen elliptischen Umriss haben kann. Jedenfalls ist der Bettschnitt 38 durch einen Seitenschnitt 40 ergänzt, welcher längs eines Teilumfangs des Bettschnitts 38 verläuft und sich - örtlich betrachtet - ausgehend von dem Bettschnitt 38 zur Korneaoberfläche hin erstreckt. Auch der Seitenschnitt 40 wird im eingeebneten Zustand der Kornea erzeugt, d.h. bei an der Kontaktfläche 26 anliegendem Auge 22, und verläuft örtlich von dem Bettschnitt 38 schräg nach außen. Alternativ kann der Seitenschnitt 38 örtlich von dem Bettschnitt 38 schräg nach innen verlaufen.

Durch den Bettschnitt 38 und den Seitenschnitt 40 zusammen wird der Flap gebildet. Dieser ist in den Figuren 2a und 2b sowie in Figur 3 mit 42 bezeichnet. In dem von dem Seitenschnitt 40 nicht umfassten Teil des Umfangs des Bettschnitts 38 hängt der Flap 42 noch mit dem übrigen Korneagewebe zusammen (abgesehen vom Bereich eines noch zu erläuternden Hilfsschnitts). Der Übergangsbereich zwischen dem Flap 42 und dem restlichen Korneagewebe bildet ein Scharnier (Hinge), das es erlaubt, den Flap wegzuklappen, um das darunter liegende Gewebe für eine ablatierende Laserbehandlung freizulegen. Die Scharnierlinie ist zumindest in ausreichender Näherung geradlinig und ist in den Fig. 2a und 2b mit 44 bezeichnet. Im Fall der Fig. 2a liegt sie näherungsweise in Überdeckung mit der geraden Kante eines Kreissegments, an dem der Bettschnitt 38 endet, in Fig. 2b verläuft sie - zumindest bei Betrachtung in der Draufsicht dieser Figur - quer über den Bettschnitt 38 hinweg von einem Umfangsende des Seitenschnitts 40 zum anderen.

Figur 3 veranschaulicht die beiden Teilschnitte (Bettschnitt, Seitenschnitt) des Flaps 42 für den Fall der Figur 2a bei entspannter Kornea, d.h. nach Wegnahme des Auges 22 von dem Kontaktelement 24. Die gestrichelt eingezeichnete Bogenlinie 48 bezeichnet die Korneavorderfläche.

Beim Schneiden des Flaps 42 entstehen infolge der Verdampfung kornealen Gewebes Gase, die aus der Schnittfläche in die angrenzenden Gewebebereiche diffundieren können. Der Verbleib solcher Gase im Auge kann einerseits gefährlich sein, wenn die Gase in besonders sensible Augenbereiche vordingen, andererseits kann er die Funktionsfähigkeit eines Eye-Trackers bei der anschließenden Laserablation des stromalen Betts beeinträchtigen. Deshalb beschränkt sich die in der Kornea erzeugte Schnittfigur nicht nur auf den Flapschnitt, sondern weist einen zusätzlichen Hilfsschnitt 50 auf, der ein Entweichen der bei der Flap-Präparation entstehenden Gase aus dem Auge ermöglicht. In den gezeigten Beispielfällen der Figuren 2a, 2b grenzt der Hilfsschnitt 50 unmittelbar an den Bettschnitt 38 an, und zwar im Scharnierbereich des Flaps 42, also dort, wo der Seitenschnitt 40 einen Teil des Umfangs des Bettschnitts 38 freilässt. Ausgehend von dem Bettschnitt 38 erstreckt sich der Hilfsschnitt 50 örtlich weg von dem Flap 42 in Richtung zur Korneaoberfläche 48, d.h. er verläuft jenseits des Flaps 42. Dabei verläuft der Hilfsschnitt 50 örtlich in zunehmend geringerer Tiefe innerhalb der Kornea, insbesondere steigt er stetig in höhere Korneaschichten an, bis er zur Korneaoberfläche gelangt. Er bildet so einen Kanal (Tunnel), durch den der Bettschnitt 38 mit der Umgebung außerhalb des Auges in Verbindung steht, so dass Gase, die beim Schneiden des Bettschnitts 38 entstehen, durch den Kanal hindurch nach außen entweichen können.

Wie in den Figuren 2a, 2b erkennbar, besitzt der von dem Hilfsschnitt 50 gebildete Kanal in den gezeigten Beispielfällen über seine Länge gleichbleibende Breite, wobei er schmäler als der Scharnierbereich des Flaps 42 ist und - bezogen auf die Richtung der Scharnierachse 44 - etwa mittig in dem Scharnierbereich liegt. Es versteht sich, dass der Hilfsschnitt 50 auch ähnlich breit wie der Scharnierbereich oder sogar breiter als dieser sein kann. Diesbezügliche Beschränkungen sind im Rahmen der Erfindung nicht beabsichtigt.

In Figur 3 ist der Hilfsschnitt 50 als geradliniger Schnitt dargestellt. Es versteht sich, dass der Hilfsschnitt 50 alternativ in einer gekrümmten Bahn örtlich vom Bettschnitt 38 zur Korneaoberfläche aufsteigen kann. Auch die Stärke des Anstiegs des Hilfsschnitts 50 kann unterschiedlich festgelegt werden. Vergleichbares gilt für die Breite des Hilfsschnitts 50; diese kann über die Länge des Hilfskanals variieren, beispielsweise in Richtung zur Korneaoberfläche hin größer werden.

Die Stelle, an welcher der Hilfsschnitt 50 an die Korneaoberfläche gelangt, kann außerhalb des Einebnungsbereich 36 der Kornea liegen, wie dies in den Figuren 2a, 2b angedeutet ist, wo sich der Hilfsschnitt 50 über den Einebnungsbereich 36 hinaus nach außen erstreckt. Es ist gleichwohl ebenso möglich, dass der Hilfsschnitt 50 innerhalb des Einebnungsbereichs 36 oder am Rand des Einebnungsbereichs örtlich an die Korneaoberfläche herangeführt ist.

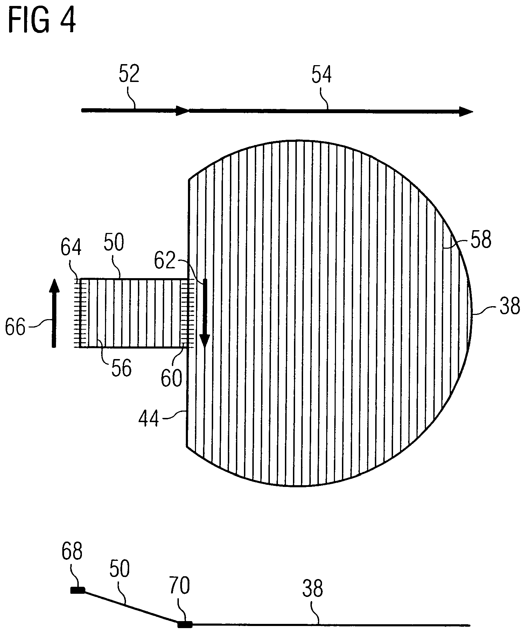

Zur Erläuterung der zeitlichen Reihenfolge, in welcher der Hilfsschnitt 50 und der Bettschnitt 38 angebracht werden und welche Scanmuster dabei für den Laserstrahl 14 verwendet werden, wird nun zusätzlich auf Figur 4 verwiesen. Dort sind der Bettschnitt 38 und der Hilfsschnitt 50 gezeigt; der Seitenschnitt 40 ist der Übersichtlichkeit halber weggelassen; er wird regelmäßig erst nach dem Bettschnitt 38 erzeugt, und zwar ausgehend von dem Bettschnitt 38 in Richtung hin zur Korneaobertläche.

Der Hilfsschnitt 50 hingegen wird erzeugt, bevor der Bettschnitt 38 angebracht wird. Dies gewährleistet, dass bereits zu Beginn der Präparation des Bettschnitts 38 Gase über den Hilfsschnitt 50 nach außen entweichen können. Der Hilfsschnitt 50 wird von der Korneaoberfläche her erzeugt, also in Richtung hin zu tieferen Korneaschichten. Dies ist durch einen in Figur 4 oben eingezeichneten Pfeil 52 angedeutet. Nach Erzeugung des Hilfsschnitts 50 wird der Bettschnitt 38 erzeugt, und zwar beginnend im Scharnierbereich 44, also dort, wo der Hilfsschnitt 50 endet. Ausgehend von dem Scharnierbereich 44 wird der Bettschnitt 38 nach und nach in Richtung zum scharnierfernen Ende hin erzeugt. Diese Erzeugungsrichtung des Bettschnitts 38 ist in Figur 4 oben durch einen Pfeil 54 angedeutet.

Auf seinem größten Teil wird der Hilfsschnitt 50 durch Linienscans des Laserstrahls 14 erzeugt, welche Linie für Linie in Pfeilrichtung 52, d.h. in Richtung von der Korneaoberfläche hin zum Bettschnitt 38, aufeinanderfolgen. Die einzelnen Scanlinien dieses Linienscans sind mit 56 bezeichnet. Auch der Bettschnitt 38 wird mit einem aus Linienscans bestehenden Scanmuster erzeugt, wobei die einzelnen Scanlinien in Richtung von dem Scharnierbereich 44 hin zum scharnierfernen Ende des Bettschnitts 38, d.h. in Pfeilrichtung 54, aufeinanderfolgen. Die Scanlinien des Bettschnitts 38 sind in Figur 4 mit 58 bezeichnet.

Während die Darstellung der Figur 4 ein Beispiel für eine Aufeinanderfolge der Scanlinien 56 in Richtung der Längserstreckung des Hilfsschnitts 50 ist (die Längserstreckung meint hierbei eine Erstreckung von der Korneaoberfläche zum Flapschnitt, genauer gesagt zum Bettschnitt), ist es ohne weiteres vorstellbar, den Hauptteil des Hilfsschnitts 50 mit einem Linienscan zu erzeugen, dessen Scanlinien quer zur Längserstreckung des Hilfsschnitts aufeinander folgen. Die Scanlinien eines solchen Quer-Scans verlaufen dann ähnlich den Scanlinien 60 und 64.

Der Übergangsbereich zwischen dem Hilfsschnitt 50 und dem Bettschnitt 38 wird zudem mit Linienscans präpariert, welche Linie für Linie quer zur Übergangsrichtung voranschreiten. Die Übergangsrichtung meint hier die Richtung, in welcher der Hilfsschnitt 50 in den Bettschnitt 38 übergeht. Diese Richtung entspricht der Richtung der Pfeile 52, 54. Durch Nebeneinandersetzen von Scanlinien quer zu dieser Richtung im Übergangsbereich gelingt es, eine gute, für den Gasdurchtritt offene Verbindung zwischen dem Hilfsschnitt 50 und dem Bettschnitt 38 zu realisieren. Die im Übergangsbereich gesetzten Quer-Scanlinien sind in Figur 4 mit 60 bezeichnet. Die Richtung ihrer Aufeinanderfolge ist durch einen Pfeil 62 dargestellt (kann wahlweise auch in umgekehrter Pfeilrichtung sein).

Ähnliche Quer-Scanlinien werden darüber hinaus im Eintrittsbereich des Hilfsschnitts 50 gesetzt, also dort, wo er an der Oberfläche der Kornea in das korneale Gewebe eintritt. Die entsprechenden Scanlinien sind in Figur 4 mit 64 bezeichnet; die Richtung ihrer Aufeinanderfolge ist durch einen Pfeil 66 angedeutet (kann wahlweise auch in umgekehrter Pfeilrichtung sein). Diese quer zur Eintrittsrichtung des Hilfsschnitts verlaufenden Scanlinien 64 sind zweckmäßig, um eine saubere Öffnung des Hilfsschnitts 50 an der Korneaoberfläche zu schaffen.

Was den zeitlichen Ablauf anbelangt, werden zweckmäßigerweise zunächst die Scanlinien 64 gesetzt, anschließend die Scanlinien 56, daraufhin die Scanlinien 60 und im Anschluss die Scanlinien 58. Auf diese Weise schreitet die Schnitterzeugung zunehmend von der Oberfläche in Richtung zu tieferen Schichten fort. Die Darstellung im unteren Teil der Figur 4 verdeutlich dies noch einmal. Dort sind der Hilfsschnitt 50, der Bettschnitt 38 sowie der Eintrittsbereich (bezeichnet mit 68) und der Übergangsbereich zwischen den beiden Schnitten (bezeichnet mit 70) in einer Ansicht von der Seite her gezeigt. Mit der erläuterten zeitlichen Abfolge der Scanlinien gelingt es, dass während der Erzeugung des Hilfsschnitts 50 und auch während der Erzeugung des Bettschnitts 38 stets bereits ein Tunnel nach außen offen ist, durch den aktuell entstehende Gase entweichen können.

In einer Abwandlung des obigen Ablaufs kann die Erzeugung des Übergangsbereichs 70 zeitlich vorgezogen werden und vor dem Eintrittsbereich 68 vorgenommen werden. Danach werden wie zuvor der Rest (d.h. der Hauptteil) des Hilfsschnitts 50 sowie der Bettschnitt 38 angebracht. In einer weiteren Abwandlung kann zunächst der Übergangsbereich 70 erzeugt werden. Sodann wird der Hauptteil des Hilfsschnitts 50 und danach der Eintrittsbereich 68 erzeugt. Nach vollständiger Erzeugung des Hilfsschnitts 50 wird der Bettschnitt 38 angebracht. Es ist freilich darauf hinzuweisen, dass im Rahmen der Erfindung keinerlei Beschränkung auf eine bestimmte zeitliche Reihenfolge der Schnitterzeugung beabsichtigt ist.

Grundsätzlich kann im gesamten Hilfsschnitt 50 (einschließlich des Eintrittsbereichs 68 und des Übergangsbereichs 70) sowie im Bettschnitt 38 der örtliche Abstand der längs der Scanlinien aufeinander folgenden Photodisruptionen im wesentlichen gleich sein. Das gleiche gilt für den gegenseitigen Abstand aufeinanderfolgender Scanlinien.

Es ist jedoch möglich, den örtlichen Abstand der Photodisruptionen oder/und den gegenseitigen Linienabstand zumindest in Teilen des Hilfsschnitts 50 oder/und des Bettschnitts 38 zu variieren. Insbesondere ist es vorstellbar, für den Eintrittsbereich 68 oder/und den Übergangsbereich 70 eine engere örtliche Aufeinanderfolge der Photodisruptionen zu wählen oder/und einen engeren gegenseitigen Abstand der aufeinanderfolgenden Scanlinien zu wählen als für den Hauptteil des Hilfsschnitts 50 und den Bettschnitts 38.

Die hier beschriebene Lage des Hilfsschnitts 50 relativ zum Bettschnitt 38 gewährleistet, dass sich die beiden Schnitte einander nicht wechselseitig überdecken. Durch eine bereits geschnittene Ebene hindurch kann nämlich keine weitere, darunterliegende Ebene geschnitten werden. Da der Hilfsschnitt 50 im Idealfall schon vorhanden sein sollte, wenn mit dem Schneiden des Bettschnitts 38 begonnen wird, ist es empfehlenswert, den Hilfsschnitt 50 von außerhalb des Flaps her (weiter vom kornealen Zentrum entfernt) in die Kornea zu schneiden und im Scharnierbereich des Flaps in den Bettschnitt übergehen zu lassen.

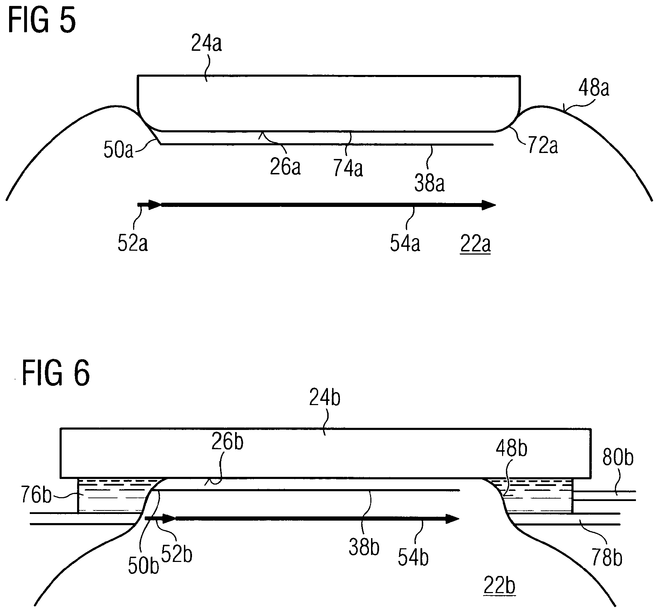

In den Figuren 5 und 6 sind gleiche oder gleichwirkende Elemente mit gleichen Bezugszeichen wie in den vorangehenden Figuren bezeichnet, jedoch ergänzt durch einen Kleinbuchstaben. Soweit sich nachstehend nichts anderes ergibt, wird zur Erläuterung dieser Elemente auf das vorstehend Gesagte verwiesen.

Es wurde bereits erläutert, dass der Hilfsschnitt innerhalb oder außerhalb des eingeebneten Bereichs der Kornea zur Oberfläche des Auges ausmünden kann. Diese Aussage kann insoweit verallgemeinert werden, als der Hilfsschnitt zur Korneaoberfläche an einer Stelle ausmünden kann, die innerhalb oder außerhalb (oder am Rand) eines Bereichs liegt, in welchem die Kornea an dem Kontaktelement des Patientenadapters anliegt. Wenngleich im Rahmen der Erfindung eine weitestgehende Einebnung der Korneaoberfläche durch das Kontaktelement angestrebt wird, ist es dennoch nicht notwendig, dass die Kornea ausschließlich in ebenen Bereichen der Kontaktfläche an dieser anliegt.

Diesbezüglich wird nun auf die Variante der Figur 5 verwiesen. Dort ist ein Kontaktelement 24a gezeigt, das auf seiner augenzugewandten Unterseite eine Kontaktfläche 26a trägt, die in ihrem Hauptteil eben ausgeführt ist, jedoch in ihrem Randbereich abgerundet ist und dort schräg zu dem ebenen Hauptteil in Richtung von dem Auge 22a weg verläuft. Der gerundete Flächenabschnitt bildet ein den ebenen Hauptteil der Kontaktfläche 26 umschließendes Ringsegment und ist mit 72a bezeichnet. Der ebene Hauptteil der Kontaktfläche 26a ist dagegen mit 74a bezeichnet. Der Kontakt zwischen dem Kontaktelement 24a und dem Auge 22a wird so hergestellt, dass sich die Kornea nicht nur in dem Hauptteil 74a sondern auch in dem äußeren Ringsegment 72a an die Kontaktfläche 26a anschmiegt.

Der Hilfsschnitt 50a wird so erzeugt, dass er im Bereich des Ringsegments 72a zur Korneaoberfläche austritt (der Begriff des Austretens ist hier rein örtlich gemeint und enthält keinerlei Angabe über die zeitliche Reihenfolge, in welcher die einzelnen Teile des Hilfsschnitts präpariert werden). Das heißt, der Hilfsschnitt 50a mündet an einer Stelle der Korneaoberfläche aus, wo die Kornea an dem rundlichen Ringsegment 72a anliegt. Dies ist insofern günstig, als etwaige Gase, die bei der Präparation des Hilfsschnitts 50a und des Bettschnitts 38a entstehen und durch den Hilfsschnitt 50a nach außen entweichen, zumindest größtenteils in die umgebende Luft gelangen können und nicht durch Kapillarwirkung tiefer zwischen das Kontaktelement 24a und die Korneaoberfläche 48a gesogen werden. Mit anderen Worten ist so eine bessere Entgasung des Operationsgebiets möglich.

Das abgerundete Ringsegment 72a schließt vorzugsweise knickfrei an den ebenen Hauptteil 74a der Kontaktfläche 26a an. Es soll dennoch nicht grundsätzlich ausgeschlossen sein, das Ringsegment 72a statt mit einer rundlichen Konfiguration als geradlinige Schrägfläche auszuführen, die durch einen Knick von dem ebenen Hauptteil 74a getrennt ist.

Die Variante der Figur 6 veranschaulicht ein Beispiel, bei dem der Hilfsschnitt 50b an einer Stelle der Korneaoberfläche ausmündet, wo die Kornea nicht an der Kontaktfläche 26b des Kontaktelements 24b anliegt. Stattdessen mündet sie außerhalb des applanierten Bereichs der Korneaoberfläche 48b aus. Konkret mündet im Beispielfall der Figur 6 der Hilfsschnitt 50 in einen Ringraum 76b aus, welcher zwischen dem Kontaktelement 24b, der Korneaoberfläche 48b und einer Dichtkomponente 78b begrenzt ist, die beispielsweise Teil eines auf das Auge 22b aufzusetzenden Saugrings (nicht näher dargestellt) sein kann. Schematisch dargestellt ist in Figur 6 ein Füllkanal 80b, über welchen der Ringraum 76b mit einer physiologischen Flüssigkeit (z.B. Kochsalzlösung) befüllbar ist. Der Füllkanal 80b kann Teil des erwähnten Saugrings sein.

Der Bettschnitt 38b und der Hilfsschnitt 50b können bei dieser Variante in einer gemeinsamen Ebene erzeugt werden, d.h. mit konstanter z-Position des Strahlfokus der für die Schnitterzeugung verwendeten Laserstrahlung. Die Erzeugungsrichtung entspricht derjenigen der Figur 4, d.h. der Hilfsschnitt 50b wird vor dem Bettschnitt 38b erzeugt, und zwar zweckmäßigerweise in einer Richtung von der Korneaoberfläche zum Bettschnitt 38b.

## Patentansprüche

1. Einrichtung zur schneidenden Bearbeitung der humanen Kornea mit fokussierter gepulster Laserstrahlung, mit steuerbaren Komponenten (16) zur Ortseinstellung des Strahlungsfokus, einem Steuerrechner (30) zur Steuerung dieser Komponenten und einem Steuerprogramm (34) für den Steuerrechner, wobei das Steuerprogramm Instruktionen enthält, welche dazu ausgelegt sind, bei Ausführung durch den Steuerrechner die Erzeugung einer einen Flapschnitt (38, 40) umfassenden Schnittfigur in der Kornea zu bewirken, wobei der Flapschnitt ein in einem Scharnierbereich mit übrigem kornealen Gewebe zusammenhängendes korneales Deckelscheibchen definiert, welches zur Seite klappbar ist, um darunterliegende Gewebebereiche der Kornea freizulegen, **dadurch gekennzeichnet, dass** die Schnittfigur ferner einen mit dem Flapschnitt in Verbindung stehenden, örtlich bis zur Korneaoberfläche (48) führenden Hilfsschnitt (50) umfasst.

2. Einrichtung nach Anspruch 1, wobei der Hilfsschnitt (50) einen flächigen, vorzugsweise im wesentlichen ebenen Kanal bildet.

3. Einrichtung nach Anspruch 2, wobei der Kanal über seine Länge im wesentlichen gleichbleibende Breite besitzt.

4. Einrichtung nach einem der Ansprüche 1 bis 3, wobei der Hilfsschnitt (50) in dem Scharnierbereich (44) des durch den Flapschnitt (38, 40) gebildeten Deckelscheibchens mit dem Flapschnitt in Verbindung steht und sich jenseits des Deckelscheibchens örtlich zur Korneaoberfläche (48) hin erstreckt.

5. Einrichtung nach einem der Ansprüche 1 bis 4, wobei der Hilfsschnitt (50) in einem Bereich (70), in dem er in Verbindung mit dem Flapschnitt steht, größte korneale Tiefe besitzt und ausgehend von diesem Bereich örtlich in zunehmend geringerer kornealer Tiefe bis hin zur Korneaoberfläche (48) verläuft.

6. Einrichtung nach einem der Ansprüche 1 bis 5, wobei die Instruktionen des Steuerprogramms (34) ausgelegt sind, den Hilfsschnitt (50) anzubringen, bevor der Flapschnitt (38, 40) gesetzt wird.

7. Einrichtung nach einem der Ansprüche 1 bis 6, wobei die Instruktionen des Steuerprogramms dazu ausgelegt sind, zumindest einen überwiegenden Teil des Hilfsschnitts (50) in einer Richtung von der Korneaoberfläche (48) hin zum Flapschnitt (38, 40) zu erzeugen.

8. Einrichtung nach einem der Ansprüche 1 bis 7, wobei die Instruktionen des Steuerprogramms dazu ausgelegt sind, zumindest einen überwiegenden Teil des Hilfsschnitts (50) mit Linienscans (56) des Strahlungsfokus zu erzeugen, welche Linie für Linie längs einer Erstreckungsrichtung des Hilfsschnitts voranschreiten, die von der Korneaoberfläche zum Flapschnitt verläuft.

9. Einrichtung nach einem der Ansprüche 1 bis 7, wobei die Instruktionen des Steuerprogramms dazu ausgelegt sind, zumindest einen überwiegenden Teil des Hilfsschnitts_mit Linienscans des Strahlfokus zu erzeugen, welche Linie für Linie quer zu einer Erstreckungsrichtung des Hilfsschnitts voranschreiten, die von der Korneaoberfläche zum Flapschnitt verläuft.

10. Einrichtung nach einem der Ansprüche 1 bis 9, wobei der Flapschnitt einen vollständig in der Tiefe, vorzugsweise in im wesentlicher gleichbleibender Tiefe, des Korneamaterials liegenden Bettschnitt (38) sowie einen an den Bettschnitt angrenzenden, zur Korneaoberfläche (48) herausgeführten Seitenschnitt (40) umfasst, wobei die Instruktionen des Steuerprogramms dazu ausgelegt sind, den Bettschnitt zeitlich vor dem Seitenschnitt anzubringen und ihn mit Linienscans (58) des Strahlungsfokus zu erzeugen, welche Linie für Linie zunehmend in Richtung weg von dem Scharnierbereich (44, 46) des Deckelscheibchens voranschreiten.

11. Einrichtung nach einem der Ansprüche 1 bis 10, wobei die Instruktionen des Steuerprogramms dazu ausgelegt sind, in einem Übergangsbereich (70) zwischen Hilfsschnitt (50) und Flapschnitt (38, 40) Linienscans (60) des Strahlungsfokus zu bewirken, welche Linie für Linie quer zur Übergangsrichtung voranschreiten.

12. Einrichtung nach einem der Ansprüche 1 bis 11, wobei die Instruktionen des Steuerprogramms dazu ausgelegt sind, im oberflächenseitigen Endbereich des Hilfsschnitts (50) Linienscans (64) des Strahlungsfokus zu bewirken, welche Linie für Linie quer zur Eintrittsrichtung des Hilfsschnitts in die Kornea voranschreiten.

13. Einrichtung nach einem der Ansprüche 1 bis 12, ferner umfassend ein für die Laserstrahlung transparentes Kontaktelement mit einer zur Anlage am Auge bestimmten Kontaktfläche, wobei die Instruktionen des Steuerprogramms dazu ausgelegt sind, den Hilfsschnitt so zu erzeugen, dass sein an der Korneaoberfläche ausmündendes Ende in einem Bereich der Kornea liegt, in dem diese an der Kontaktfläche des Kontaktelements anliegt.

14. Einrichtung nach Anspruch 13, wobei die Kontaktfläche einen planen Flächenabschnitt zur Einebnung eines Teils der Korneaoberfläche aufweist, wobei die Instruktionen des Steuerprogramms dazu ausgelegt sind, den Hilfsschnitt so zu erzeugen, dass sein an der Korneaoberfläche ausmündendes Ende in einem Bereich der Kornea liegt, in dem diese an dem planen Flächenabschnitt der Kontaktfläche anliegt.

15. Einrichtung nach Anspruch 13, wobei die Kontaktfläche einen planen Flächenabschnitt (74a) zur Einebnung eines Teils der Korneaoberfläche sowie einen an den planen Flächenabschnitt angrenzenden, schräg zu diesem in Richtung zur augenabgewandten Seite des Kontaktelements verlaufenden Flächenabschnitt (72a) aufweist, wobei die Instruktionen des Steuerprogramms dazu ausgelegt sind, den Hilfsschnitt (50a) so zu erzeugen, dass sein an der Korneaoberfläche ausmündendes Ende in einem Bereich der Kornea liegt, in dem diese an dem schräg verlaufenden Flächenabschnitt der Kontaktfläche anliegt.

16. Einrichtung nach Anspruch 15, wobei der schräg verlaufende Flächenabschnitt (72a) gerundet ausgeführt ist und knickfrei an den planen Flächenabschnitt anschließt.

17. Einrichtung nach einem der Ansprüche 1 bis 12, ferner umfassend ein für die Laserstrahlung transparentes Kontaktelement mit einer zur Anlage am Auge bestimmten Kontaktfläche (26b), wobei die Instruktionen des Steuerprogramms dazu ausgelegt sind, den Hilfsschnitt so zu erzeugen, dass sein an der Korneaoberfläche ausmündendes Ende außerhalb eines Bereichs der Kornea liegt, in dem diese an der Kontaktfläche des Kontaktelements anliegt.

18. Einrichtung nach Anspruch 17, ferner umfassend einen auf der augenzugewandten Seite des Kontaktelements vorgesehenen, über eine Füllkanalanordnung (80b) mit einem fließfähigen Medium befüllbaren Raum (76b), wobei die Instruktionen des Steuerprogramms dazu ausgelegt sind, den Hilfsschnitt so zu erzeugen, dass er in den Raum mündet.

19. Einrichtung nach Anspruch 17 oder 18, wobei der Flapschnitt einen ebenen Bettschnitt umfasst und die Instruktionen des Steuerprogramms dazu ausgelegt sind, den Hilfsschnitt und den Bettschnitt in einer gemeinsamen Ebene zu erzeugen.

## Claims

1. Device for cutting the human cornea with focused pulsed laser radiation, having controllable components (16) for setting the position of the radiation focus, a control computer (30) for controlling these components, and a control program (34) for the control computer, the control program containing instructions which are designed such that, when executed by the control computer, they cause the generation, in the cornea, of an incision figure comprising a flap incision (38, 40), the flap incision defining a corneal flap which is contiguous with the rest of the corneal tissue in a hinge area and which can be folded to the side in order to expose underlying tissue areas of the cornea, **characterized in that** the incision figure moreover comprises an auxiliary incision (50) connected to the flap incision and leading locally as far as the corneal surface (48).

2. Device according to Claim 1, wherein the auxiliary incision (50) forms a two-dimensional, preferably substantially flat channel.

3. Device according to Claim 2, wherein the channel has a substantially constant width along its length.

4. Device according to one of Claims 1 to 3, wherein the auxiliary incision (50) is connected to the flap incision in the hinge area (44) of the flap formed by the flap incision (38, 40) and, on the other side of the flap, extends locally to the corneal surface (48).

5. Device according to one of Claims 1 to 4, wherein the auxiliary incision (50) has its greatest corneal depth in an area (70) in which it is connected to the flap incision and, starting from this area, extends locally with an increasingly smaller corneal depth as far as the corneal surface (48).

6. Device according to one of Claims 1 to 5, wherein the instructions of the control program (34) are designed to produce the auxiliary incision (50) before the flap incision (38, 40) is made.

7. Device according to one of Claims 1 to 6, wherein the instructions of the control program are designed to generate at least a predominant part of the auxiliary incision (50) in a direction from the corneal surface (48) to the flap incision (38, 40).

8. Device according to one of Claims 1 to 7, wherein the instructions of the control program are designed to generate at least a predominant part of the auxiliary incision (50) with line scans (56) of the radiation focus which advance, line by line, along a direction of extent of the auxiliary incision that runs from the corneal surface to the flap incision.

9. Device according to one of Claims 1 to 7, wherein the instructions of the control program are designed to generate at least a predominant part of the auxiliary incision with line scans of the radiation focus which advance, line by line, transverse to a direction of extent of the auxiliary incision that runs from the corneal surface to the flap incision.

10. Device according to one of Claims 1 to 9, wherein the flap incision comprises a bed incision (38) lying completely within the depth of the corneal material, preferably at a substantially constant depth, and also a lateral incision (40) adjoining the bed incision and guided out to the corneal surface (48), wherein the instructions of the control program are designed to produce the bed incision temporally before the lateral incision and to generate it with line scans (58) of the radiation focus which advance, line by line, increasingly in a direction away from the hinge area (44, 46) of the flap.

11. Device according to one of Claims 1 to 10, wherein the instructions of the control program are designed to bring about, in a transition area (70) between auxiliary incision (50) and flap incision (38, 40), line scans (60) of the radiation focus which advance line by line transverse to the transition direction.

12. Device according to one of Claims 1 to 11, wherein the instructions of the control program are designed to bring about, in the surface-side end area of the auxiliary incision (50), line scans (64) of the radiation focus which advance line by line transverse to the direction of entry of the auxiliary incision into the cornea.

13. Device according to one of Claims 1 to 12, further comprising a contact element that is transparent to the laser radiation, with a contact face intended to bear on the eye, wherein the instructions of the control program are designed to generate the auxiliary incision in such a way that its end opening out on the corneal surface lies in an area of the cornea in which the latter bears on the contact face of the contact element.

14. Device according to Claim 13, wherein the contact face has a plane surface portion for levelling a part of the corneal surface, wherein the instructions of the control program are designed to generate the auxiliary incision in such a way that its end opening out on the corneal surface lies in an area of the cornea in which the latter bears on the plane surface portion of the contact face.

15. Device according to Claim 13, wherein the contact face has a plane surface portion (74a) for levelling a part of the corneal surface, and also a surface portion (72a) adjoining the plane surface portion and extending obliquely in relation to the latter in the direction towards the side of the contact element facing away from the eye, wherein the instructions of the control program are designed to generate the auxiliary incision (50a) in such a way that its end opening out on the corneal surface lies in an area of the cornea in which the latter bears on the obliquely extending surface portion of the contact face.

16. Device according to Claim 15, wherein the obliquely extending surface portion (72a) is rounded and adjoins the plane surface portion in a manner free of kinks.

17. Device according to one of Claims 1 to 12, further comprising a contact element that is transparent to the laser radiation, with a contact face (26b) intended to bear on the eye, wherein the instructions of the control program are designed to generate the auxiliary incision in such a way that its end opening out on the corneal surface lies outside an area of the cornea in which the latter bears on the contact face of the contact element.

18. Device according to Claim 17, further comprising a chamber (76b) which is provided on the side of the contact element facing towards the eye and which is fillable with a free-flowing medium via a filling-channel arrangement (80b), wherein the instructions of the control program are designed to generate the auxiliary incision in such a way that it opens into the chamber.

19. Device according to Claim 17 or 18, wherein the flap incision comprises a flat bed incision, and the instructions of the control program are designed to generate the auxiliary incision and the bed incision in a common plane.

## Revendications

1. Dispositif destiné à traiter par découpe la cornée humaine au moyen d'un rayonnement laser focalisé et pulsé, muni de composants (16) pouvant être commandés pour le réglage de positions du foyer du rayonnement, un calculateur de commande (30) destiné à commander lesdits composants et un programme de commande (34) destiné au calculateur de commande, dans lequel le programme de commande contient des instructions qui sont conçues, lors de leur exécution par le calculateur de commande, pour provoquer la production d'une figure de découpe comprenant une découpe de volet (38, 40) dans la cornée, dans lequel la découpe de volet définit une lamelle de protection cornéenne d'un seul tenant avec le reste du tissu cornéen dans une zone de charnière qui peut être rabattue sur le côté afin de libérer des zones sous-jacentes de la cornée, **caractérisé en ce que** la figure de découpe comprend en outre une découpe auxiliaire (50) reliée à la découpe de volet et conduisant spatialement jusqu'à la surface de la cornée (48).

2. Dispositif selon la revendication 1, dans lequel la découpe auxiliaire (50) forme un canal plat, de préférence sensiblement plan.

3. Dispositif selon la revendication 2, dans lequel le canal plat présente une largeur sensiblement constante sur toute sa longueur.

4. Dispositif selon l'une quelconque des revendications 1 à 3, dans lequel la découpe auxiliaire (50) est reliée, dans la zone de charnière (44) de la lamelle de protection formée par la découpe de volet (38, 40), à la découpe de volet et s'étend spatialement de chaque côté de la lamelle de protection jusqu'à la surface (48) de la cornée.

5. Dispositif selon l'une quelconque des revendications 1 à 4, dans lequel la découpe auxiliaire (50) présente la profondeur cornéenne la plus grande dans une zone (70) dans laquelle elle est reliée à la découpe de volet et s'étend spatialement depuis ladite zone à une profondeur cornéenne progressivement croissante, jusqu'à la surface (48) de la cornée.

6. Dispositif selon l'une quelconque des revendications 1 à 5, dans lequel des instructions du programme de commande (34) sont conçues pour pratiquer la découpe auxiliaire (50) avant de réaliser la découpe de volet (38, 40).

7. Dispositif selon l'une quelconque des revendications 1 à 6, dans lequel les instructions du programme de commande sont conçues pour générer au moins une partie prédominante de la découpe auxiliaire (50) dans une direction allant de la surface cornéenne (48) vers la découpe de volet (38, 40).

8. Dispositif selon l'une quelconque des revendications 1 à 7, dans lequel les instructions du programme de commande sont conçues pour générer une partie prédominante de la découpe auxiliaire (50) par des balayages linéaires (56) du foyer du rayonnement, lesquels balayages progressent ligne par ligne le long d'une direction d'extension de la découpe auxiliaire, qui s'étend de la surface cornéenne vers la découpe de volet.

9. Dispositif selon l'une quelconque des revendications 1 à 7, dans lequel les instructions du programme de commande sont conçues pour générer au moins une partie prédominante de la découpe auxiliaire par des balayages linéaires du foyer du faisceau, lesquels balayages progressent ligne par ligne transversalement à une direction d'extension de la découpe auxiliaire, qui s'étend de la surface cornéenne vers la découpe de volet.

10. Dispositif selon l'une quelconque des revendications 1 à 9, dans lequel la découpe de volet délimite une découpe de lit (38) se situant entièrement en profondeur, de préférence à une profondeur sensiblement constante, du matériau cornéen, ainsi qu'une découpe latérale (40) délimitant la découpe du lit et allant vers la surface cornéenne (48), dans lequel les instructions du programme de commande sont conçues pour pratiquer la découpe du lit de manière à ce qu'elle précède dans le temps la découpe latérale et pour la générer par des balayages linéaires (58) du foyer du rayonnement, lesquels balayages progressent ligne par ligne en croissant dans une direction qui s'éloigne de la zone de charnière (44, 46) de la lamelle de protection.

11. Dispositif selon l'une quelconque des revendications 1 à 10, dans lequel les instructions du programme de commande sont conçues pour provoquer des balayages linéaires (60) du foyer du rayonnement dans une zone de transition (70) entre la découpe auxiliaire (50) et la découpe de volet (38, 40), lesquels balayages linéaires progressent ligne par ligne transversalement à la direction de transition.

12. Dispositif selon l'une quelconque des revendications 1 à 11, dans lequel les instructions du programme de commande sont conçues pour provoquer, dans la zone d'extrémité, du côté de la surface de la découpe auxiliaire (50), des balayages linéaires (64) du foyer du rayonnement, qui progressent ligne par ligne transversalement à la direction de pénétration de la découpe auxiliaire dans la cornée.

13. Dispositif selon l'une quelconque des revendications 1 à 12, comprenant en outre un élément de contact transparent au rayonnement laser présentant une surface de contact déterminée destinée à être appliquée à l'oeil, dans lequel les instructions du programme de commande sont conçues pour générer la découpe auxiliaire de manière à ce que son extrémité débouchant au niveau de la surface cornéenne se situe dans une zone de la cornée dans laquelle elle est adjacente à la surface de contact de l'élément de contact.

14. Dispositif selon la revendication 13, dans lequel la surface de contact présente une découpe de surface plane destinée à niveler un partie de la surface cornéenne, dans lequel les instructions du programme de commande sont conçues pour réaliser la découpe auxiliaire de manière à ce que son extrémité débouchant au niveau de la surface cornéenne se situe dans une zone de la cornée dans laquelle elle est adjacente à la découpe superficielle plane de la surface de contact.

15. Dispositif selon la revendication 13, dans lequel la surface de contact comporte une découpe de surface plane (74a) destinée à niveler une partie de la surface cornéenne, ainsi qu'une section plate (72a) adjacente à la section de surface plane, transversalement par rapport à celle-ci dans une direction orientée vers la face opposée à l'oeil de l'élément de contact, dans lequel les instructions du programme de commande sont conçues pour réaliser la découpe auxiliaire (50a) de manière à ce que son extrémité débouchant au niveau de la surface cornéenne se situe dans une zone de la cornée dans laquelle elle est adjacente à la découpe de surface de la surface de contact s'étendant transversalement à celle-ci.

16. Dispositif selon la revendication 15, dans lequel la découpe de surface s'étendant transversalement (72a) est réalisée de manière à être arrondie et est contiguë à la découpe de surface plane sans former de coude.

17. Dispositif selon l'une quelconque des revendications 1 à 12, comprenant en outre un élément de contact transparent au rayonnement laser ayant une surface de contact (26b) déterminée pour être appliquée à l'oeil, dans lequel les instructions du programme de commande sont conçues pour réaliser la découpe auxiliaire de manière à ce que son extrémité débouchant au niveau de la surface cornéenne se situe en dehors de la zone de la cornée dans laquelle elle est adjacente à la surface de contact de l'élément de contact.

18. Dispositif selon la revendication 17, comprenant en outre un espace (76b) prévu sur la face tournée vers l'oeil de l'élément de contact, pouvant être rempli d'un milieu fluide par l'intermédiaire d'un système de canaux de remplissage (80b), dans lequel les instructions du programme de commande sont conçues pour réaliser la découpe auxiliaire de manière à ce qu'elle débouche dans ledit espace.

19. Dispositif selon la revendication 17 ou 18, dans lequel la découpe de volet comprend une découpe de lit plane et les instructions du programme de commande sont conçues pour réaliser la découpe auxiliaire et la découpe de lit dans un plan commun.
